# EUROPEAN PATENT APPLICATION

(11) **EP 1 508 599 A1**
(43) Date of publication of application: **23.02.2005**
(21) Application number: 03018678.7
(22) Date of filing: 22.08.2003
(51) Int. Cl.: C09C 1/04, A61K 7/00

(54) **Surface-modified zinc oxide**

(71) Applicant: Degussa AG, 40474 Düsseldorf (DE)
(72) Inventor: Meyer, Jürgen Dr., 63811 Stockstadt (DE); Günther, Michael, 63791 Stockstadt (DE)

(57) **Abstract**

Surface-modified zinc oxides with a BET surface area of 18 ± 5 m²/g and a C content of 0.1 to 5.0 wt.% are prepared by spraying the zinc oxides with the surface-modifying agent or adding this in vapour form and then heat-treating the mixture.

They can be employed for the preparation of cosmetics.

## Description

The invention relates to surface-modified zinc oxides, a process for their preparation and their use.

One 0,portion of the solar spectrum comprises wavelengths of electromagnetic energy which range between about 290 and 3,000 nm. This range may be divided into different regions, namely:
1. the ultraviolet region (290-400 nm)
2. the visible region (400-760 nm) and
3. the near-infrared region (> 760 nm).

The ultraviolet region has, moreover, been arbitrarily divided into three bands, referred to as the UVA, UVB and UVC bands.

The UVB band extends from 290 to 320 nm. It is the principal cause of the sunburn reaction and it is also the most effective in stimulating the tanning reaction in the skin. UVC radiation (200-290 nm) from the sun does not reach the surface of the earth, although one can encounter radiation in this range from artificial sources such as germicidal lamps and high and low pressure mercury arc lamps. For purposes of the present invention however, protection against UVC radiation is generally not a major concern, i.e., in contrast to the dangers posed by UVA and UVB radiation, The UVA band, which extends from 320-400 nm, can also cause the tanning reaction. UVA radiation can also cause sunburns, but its capacity to do so is less than that of UVB radiation.

The amount of UVA radiation exposure, however, is increasing. This is due to the fact that most sunscreens effectively block only UVB radiation. As stated above, UVB radiation is more capable than UVA radiation of causing the tanning and burning reactions. Therefore, if one is using a sunscreen that blocks UVB radiation he/she will tend to stay in the sun for an extended period of time because the immediate effects of the sun tan/burn are not evident. The problem is that UVA is still penetrating the skin and although it is not causing any immediately obvious effects, it is causing long term damage. In recent years, it has been well documented that UVA radiation, like UVB radiation, is harmful to the skin. In fact, current data reveal that solar radiation containing these wavelengths is the chief cause of skin cancer, which presently accounts for 30-40 % of all new cancers each year. In the United States alone, 500,000 new cases of skin cancer will be reported this year and the number is expected to keep rising in the future. UVA radiation has been shown to promote skin cancer by inhibiting enzymes that repair cells damaged by UVB radiation. UVA radiation also penetrates more deeply into the skin than UVB radiation and causes changes in blood vessels and premature aging of the skin, thus adding to the damage produced by UVB rays (see, e.g., Hurwitz, Sidney, "The Sun and Sunscreen Protection: Recommendations for Children" Dermatol. Surg. Oncol; 14:6 (June 1988) P 657).

The goal of any sunscreen should thus be to protect the user from both UVA and UVB radiation with a minimum of side effects. This end has not been adequately achieved with the use of presently available sunscreen products.

Sunscreen products can be grouped into two broad categories, i.e.,
1. topical sunscreens and
2. oral sunscreens.

The present invention focuses upon the topical sunscreens, which can be further differentiated into two subcategories, namely
1. chemical sunscreens and
2. physical sunscreens.

Chemical sunscreens contain from about 3 to about 26 % of one or more UV-absorbing chemicals. When applied to the surface of the skin as a thin film, i.e., about 10-15 µm in thickness, these chemicals act as a filler to diminish the penetration of UV radiation to the cells of the epidermis.

These sunscreens are typically applied in a cream, oil, lotion, alcohol or gel vehicle and they are usually colorless, because they do not contain any visibly light-absorbing chemicals.

The most widely-used chemical sunscreens contain, for example, para-aminobenzoic acid (PABA), PABA esters (glyceryl PABA), amyldimethyl PABA and octyldimethyl PABA), benzophenones (oxybenzone and sulisobenzone), cinnamates (octylmethoxy cinnamate and cinoxate), salicylates (homomethyl salicylate) and anthranilates.

To date, more than twenty-one such chemicals have been approved by the United States Food and Drug Administration as "safe and effective" agents in protecting skin against sunburn (see, e.g., Pathak, Madhu, "Sunscreens: Topical and Systemic Approaches for Protection of Human Skin Against Harmful Effects of Solar Radiation", Continuing Medical Education Series, J. Am. Acad. Dermat., 7:3 (September 1982) p. 285, 291).

Questions have recently been raised, however, by the medical profession as to whether the chemical components of these sunscreens are indeed inert and further, whether repeated use of such sunscreens can result in significant transdermal absorption of these chemicals. Because chemical sunscreens are applied topically in relatively high concentrations (i.e., up to 26 %), contact and photocontact sensitization can occur, as well as hypersensitivity, i.e., photoallergic reactions (see Drumgoogle et al., "Sunscreening Agent Intolerance: Contact and Photocontact Sensitization and Contact Urticania" J. Am. Acad. Dermatol., 1990:22, p. 1068).

Physical sunscreens, on the other hand, comprise particles of a relatively physiologically inert sunblock, i.e., UV-absorbing, compound typically suspended in a cream or lotion. Materials frequently utilized for this purpose include kaolin, talc and two metal oxides, i.e., titanium dioxide and zinc oxide. The latter two compounds are not associated with the inflammatory reactions noted above.

The physical sunscreen products are, however, typically messy and occlusive. Moreover, they additionally form a visible, colored (e.g., white) layer on the surface of the skin, which is cosmetically unacceptable to many that are in need of sunscreen protection. This causes many such individuals to forego the use of these products. The color of these compositions is attributable to the optical properties of the particles from which these materials are formed. These properties are at least partially dependent upon the size of these particles, which typically have a fairly "standard" range of diameters, measured in tenths of a micron (i.e., about greater than about 0,7-0,7µ).

In addition, presently available physical sunscreens are not easily washed off of the user's body. Instead, they typically melt off with the heat of the sun, thus incidentally staining or otherwise discoloring the user's clothing. Moreover, because they are applied as relatively thick films (20-50 µm), use of these products may also promote undesirable skin conditions, including miliaria, a skin disease caused by an inflammation of the sweat glands, and folliculitis, an inflammation of the hair follicle, As such, these physical sunscreen products are deemed cosmetically unacceptable by a large class of image conscious persons, which primarily includes young people. Unfortunately, this same group is the exact population that needs solar protection the most.

It has stated that proper use of sunscreens prior to the age of 18 would prevent 80 % of skin cancers (see e.g., Taylor et al., "Photoaging/Photodamage and Photoprotection" 22 J. Am. Acad. Dermatol., 9 (1990).

In one variant of the "typical" prior art physical sunblocks described above, certain commercial sunscreen products containing titanium dioxide are made with what is known as "micronized" or "large surface area" particles of the metal oxide.

It should be noted here that the term "micronized" does not denote a specific particle size. Rather, the term is only used to describe small particles having a large surface area. The titanium dioxide particles utilized in these sunblock products have a diameter an order of magnitude smaller (i.e., measuring about 0,01 µ) than the "standard" sized particles (measuring about greater than about 0,7-0,9 µ) described above.

One drawback to the use of this material, however, is that titanium dioxide absorbs neither as much UV-radiation nor transmits as much visible radiation as, for example, zinc oxide, which is utilized by applicants in the present invention (see, e.g., Brown, Harvey E., Zinc Oxide: Properties and Applicants, pp. 11-12, FIG. 2-4 (1976)). Thus, although the use of micronized titanium dioxide particles does render the resultant product smoother and less occlusive, it does not obviate the main drawback faced with the use of this material, i.e., its comparatively lower effectiveness (in contrast to ZnO) as a sunblock agent.

Titanium dioxide-based products are also more opaque than those formed with the zinc oxide of the present invention, which is due to the fact that the crystalline structure of the titanium dioxide material renders it only partially transparent to visible wavelengths of light and thus not generally as acceptable for cosmetic use.

Although it has been known to form micronized particles of zinc oxide for very specialized uses in the rubber industry, these particles contain substantial quantities (i.e., greater than about 200 ppm) of trace metals such as lead, mercury, arsenic and cadmium. The potential dangers to human health caused by exposure to these materials is well documented.
Thus, such zinc oxide particles containing these levels of trace metals are not acceptable for topical application to human skin.

Greater public awareness of the harmful effects of exposure to excessive solar radiation has therefore resulted in an increased use of sunscreen products by the public, coupled with a call for improved sunscreen materials free of the drawbacks described above by those whose livelihood and/or leisure activities cause them to be exposed to any substantial amounts of solar radiation.

To avoid these problems a topical formulation for shielding skin from ultraviolet radiation is known which comprises:
- a substantially colorless dermatologically acceptable liquid carrier;
- micronized particles of zinc oxide, said particles having an average particle diameter of less than about 0,2 microns and containg:
   lead < 20 ppm;
   arsenic < 3 ppm;
   cadmium < 15 ppm; and
   mercury < 1 ppm
   said particles being substantially uniformly dispersed in said substantially colorless dermatologically acceptable liquid carrier to form a substantially visibly transparent topical sunblock formulation, said particles being dispersed in said carrier in an amount effective to shield skin over which said substantially visibly transparent topical sunblock formulation is applied from hazardous effects of UVA and UVB radiation.

Zinc oxide is a reactive material which exhibits a wide range of reactivity with alkaline as well as acidic solutions, liquids and gases. In some applications the reactive nature of the zinc oxide is desirable, for example in paint applications, the reactivity of the pigment results in adhesion into the polymer film. In many applications, it is highly desirable to have zinc oxide in a non-reactive form, that is to eliminate, or make unavailable, the active sites present on the molecule.

Harvey Brown in his book Zinc Oxide Properties and Applications (International Lead Zinc Research Organization) states zinc oxide displays a high degree of reactivity in water with a wide range of materials, including acids, acid salts, and alkaline materials. Many of the resulting compounds are complex structures because of the variety of species furnished by zinc oxide in aqueous solution. Brown goes on to state that zinc oxychloride, zinc phosphates, zinc silicates, and a variety of other materials can be formed in aqueous media.

One measure of the availability of reactive groups on the zinc oxide is pH change associated with use of zinc oxide. Zinc oxide containing reactive sites can increase the pH of aqueous products. In some instances the increase can be from an initial pH of 7 to a pH of 8,7. This increase is not only a measure of the presence of reactive groups, but is highly undesirable in the formulation.

It is therefore very desirable to produce a zinc oxide, which has the pigment properties but lacks the reactivity found in untreated zinc oxide.

One area in which zinc oxide has been used is in sunscreen products. It protects the skin from sun.
The traditional materials used for protecting the skin from the harmful effect of the sun are the organic sunscreens. These include para amino benzoic acid and other materials, which absorb ultra violet light.
Recently, studies have indicated that ultra violet light is a major factor in the ageing of skin. This has resulted in the incorporation of sunscreens in products, which are not aimed specifically for use at the beach, like make up. Additionally, there has been an increased interest in providing higher levels of protection to the skin.

The called SPF system has been developed to evaluate various materials for their effectiveness in protecting the skin from the damaging affects of the sun. The quest for higher and higher SPF values has resulted in the use of greater levels of organic sunscreen. These materials have a tendency to be irritating at high concentrations, and have the affect of increasing the available organic material for bacteria. This result in the need for more preservative to protect the higher level of organic sun screen agent from bacterial degradation. The higher levels of preservative result in higher irritation levels, which can be addressed by incorporation of irritation mitigates, which themselves are degraded by bacteria.

The use of inorganic sunscreen agents like zinc oxide is a good way around the use of organic sunscreens, since they are not attacked by bacteria. However, their use does have some other inherent problems. Specifically, these materials are not easily formulated into stable products, due to the reactivity issues raised above. Zinc oxide tends to agglomerate in many finished formulations, loosing it's effectiveness in the formulation and resulting in unacceptable aesthetic results, most commonly whitening and viscosity changes. Additionally, zinc oxide tends to raise the pH of the formulation to about 8,5, which is too high for many skin care formulations. These formulations tend to be useful at a pH of 6-7. Zinc oxide has limited usefulness as is due to these problems.

One approach has been to pre-disperse the zinc oxide in an organic oil like Siltech's patented tri(octyldodecyl)citrate. While the dispersion is fairly stable, the coating is not permanent since there is no reaction between the oil and the zinc oxide. The oil also disrupts the uniformity of the zinc oxide on the skin. Traditionally, dispersing aids have been added to formulations to minimize the disruptive effect upon the film. These include phosphate esters, and lecithin. These too suffer from the labile nature of the surface treatment and dissociation between the particle and the oil. This is especially evident when zinc oxide is exposed to extreme mechanical or thermal stress as in the production of plastics or stick cosmetics.

It is known to overcome the shortfalls of zinc oxide by reacting a specific silicone compound under controlled conditions to produce a stable, surface treated zinc oxide which maintains it's state of dispersions and does not contribute significantly to chemical instability in the formulations.

According to US 5,486,631 it has been found that highly effective system for hydrophobizing zinc oxide makes use of a silicone compound conforming to the following structure: 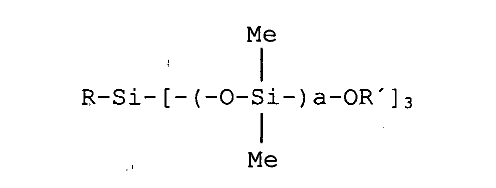
Me is methyl;
R is alkyl having one to ten carbon atoms;
R' is methyl or ethyl;
a is an integer ranging from 4 to 12.

The known process for hydrophobizing zinc oxide and the resultant hydrophobic zinc oxide show the disadvantage that the hydrophobazing agent produces a polymerized cover on the surface of the zinc oxide.

It is one object of the invention to overcome the disadvantage of the known hydrophobic zinc oxide.

The invention provides surface-modified zinc oxides, which are characterized in that they have the following physico-chemical characteristic data:

| | |
|---|---|
| BET surface area: | 18 ± 5 m²/g |
| C content | 0.1 to 5.0 wt.% |

The surface-modified zinc oxides according to the invention can furthermore have a loss on drying of 0.1 to 0.2% and a loss on ignition of 0.8 to 1.4.

The surface-modified zinc oxide according to the invention' preferably has defined molecular groups on the surface.

The invention also provides a process for the preparation of the surface-modified zinc oxides according to the invention, which is characterized in that the zinc oxides, optionally after spraying with water, are sprayed with the surface-modifying agent at room temperature and the mixture is then heat-treated at a temperature of 50 to 400°C over a period of 1 to 6 h.

Alternatively, the surface-modified zinc oxides according to the invention can be prepared by treating the zinc oxides, optionally after spraying with water, with the surface-modifying agent in vapour form and then heat-treating the mixture at a temperature of 50 to 800°C over a period of 0.5 to 6 h.

The heat treatment can be carried out under an inert gas, such as, for example, nitrogen.
The surface modification can be carried out continuously or batchwise in heatable mixers and dryers with spray devices. Suitable devices can be, for example: plough share mixers or plate, fluidized bed or flow-bed dryers.

Any desired zinc oxide can be employed as the hydrophilic zinc oxide. For example, a zinc oxide which is known from WO 92/13517 can be employed. A zinc oxide which is described in the earlier Application according to DE 102 12 680 can preferably be employed.

This zinc oxide is a nanoscale, pyrogenically produced zinc oxide powder having a BET surface area of 10 to 200 m²/g, characterised in that it is in the form of aggregates of anisotropic primary particles and that the aggregates display an average diameter of 50 to 300 nm.

The primary particles are understood to be the smallest particles in high-resolution TEM images, which are obviously unable to be broken down any further. Several primary particles can congregate at their points of contact to form aggregates. These aggregates are either impossible or very difficult to break down again using dispersing devices. Several aggregates can join together loosely to form agglomerates, whereby this process can be reversed again by suitable dispersion.

The term anisotropic means that the arrangement of atoms differs along the three spatial axes. Anisotropic primary particles include for example those that are acicular, nodular or platelet-shaped. A cubic or spherical arrangement, for example, would be isotropic.

Pyrogenic refers to the formation of oxides by flame oxidation of metals or non-metals or compounds thereof in the gas phase in a flame produced by reaction of a fuel gas, preferably hydrogen, and oxygen. Highly disperse, non-porous primary particles are initially formed which, as the reaction continues, coalesce to form aggregates, and these can congregate further to form agglomerates.

In a particular embodiment the aggregates can comprise a mixture of nodular primary particles and acicular primary particles, whereby the ratio of nodular to acicular primary particles can be between 99:1 and 1:99.

The nodular primary particles preferably display an average diameter of 10 to 50 nm and the acicular primary particles preferably display a length of 100 nm to 2000 nm and a width of 10 nm to 100 nm.

The aggregates in the powder can display a largely anisotropic structure, defined by a shape factor F(circle) of below 0.5. The variable F(circle) describes the deviation of an aggregate from a perfect circular shape. In a perfect circular object F(circle) equals 1. The lower the value, the further removed the object structure from the perfect circular shape. The parameter is defined according to ASTM 3849-89.

The powder can display at its surface an oxygen concentration as non-desorbable moisture in the form of Zn-OH and/or Zn-OH₂ units of at least 40%. It is determined by XPS analysis (XPS = X-ray photoelectron spectroscopy) of the oxygen signals at 532 to 533 eV and 534 to 535 eV.

The powder can preferably display a transmission of no more than 60% at a wavelength of 310 nm and 360 nm.

In a particular embodiment the bulk density of the powder is 40 to 120 g/l.

Te production of the powder is characterised in that zinc powder is converted into zinc oxide powder in four successive reaction zones, evaporation zone, nucleation zone, oxidation zone and quench zone,
◆ whereby in the evaporation zone the zinc powder conveyed there by an inert gas stream is evaporated in a flame of air and/or oxygen and a fuel gas, preferably hydrogen, under the proviso that the reaction parameters are chosen such that oxidation of the zinc does not occur,
◆ and whereby in the nucleation zone, where the hot reaction mixture, consisting of zinc vapour, water vapour as a reaction product of the flame reaction and optionally excess fuel gas, arrives from the evaporation zone, it cools to temperatures below the boiling point of zinc or is cooled by means of an inert gas,
◆ and whereby in the oxidation zone the mixture from the nucleation zone is oxidised with air and/or oxygen,
◆ and whereby in the quench zone the oxidation mixture is cooled to temperatures of below 400°C by addition of cooling gas (for example nitrogen, air, argon, carbon dioxide).

The process can be performed in such a way that in the evaporation zone an excess of fuel gas is used, expressed in lambda values of 0.5 to 0.99, preferably 0.8 to 0.95.

In a particular embodiment the process can be performed in such a way that the temperature in the evaporation zone is preferably between 920 °C and 2000°C. In the nucleation zone the temperature can preferably be between 500°C and 900°C, particularly preferably between 700°C and 800°C.

Furthermore the cooling rate
◆ in the nucleation zone can preferably be between 100 Kelvin/seconds and 10000 Kelvin/seconds, particularly preferably between 2000 Kelvin/seconds and 3000 Kelvin/seconds and
◆ in the quench zone the cooling rate can preferably be between 1000 Kelvin/seconds and 50000 Kelvin/seconds, particularly preferably between 5000 Kelvin/seconds and 15000 Kelvin/seconds.

The residence time of the reaction mixture in the
◆ evaporation zone can preferably be between 0.1 seconds and 4 seconds, preferably between 0.5 seconds and 2 seconds,
◆ in the nucleation zone between 0.05 seconds and 1.00 seconds, preferably between 0.1 seconds and 0.2 seconds,
◆ in the oxidation zone between 5 milliseconds and 200 milliseconds, preferably between 10 milliseconds and 30 milliseconds,
◆ and in the quench zone between 0.05 seconds and 1.00 seconds, preferably between 0.1 seconds and 0.2 seconds.,

The process can also be performed in such a way that air and/or oxygen and the fuel gas can be supplied to one or more points within the evaporation zone.

The zinc oxide powder can be separated from the gas stream by means of a filter, cyclone, washer or other suitable separators.

The following compounds can be employed as the surface-modifying agent:
a) Organosilanes of the type (RO)₃Si(CₙH₂ₙ₊₁) and (RO)₃Si(CₙH₂ₙ₋₁)
   - R =: alkyl, such as, for example, methyl-, ethyl-, n-propyl-, i-propyl-, butyl-
   - n =: 1 - 20
b) Organosilanes of the type R'ₓ(RO)_{y}Si(CₙH₂ₙ₊₁) and R'x(RO)ySi(CₙH₂ₙ₋₁)
   - R =: alkyl, such as, for example, methyl-, ethyl-, n-propyl-, i-propyl-, butyl-
   - R' =: alkyl, such as, for example, methyl-, ethyl-, n-propyl-, i-propyl-, butyl-
   - R'=: cycloalkyl
   - n =: 1 - 20
   - x+y =: 3
   - x =: 1,2
   - y =: 1,2
c) Halogeno-organosilanes of the type X₃Si(CₙH₂ₙ₊₁) and X₃Si(CₙH₂ₙ₋₁)
   - X =: Cl, Br
   - n =: 1 - 20
d) Halogeno-organosilanes of the type X₂(R')Si(CₙH₂ₙ₊₁) and X₂(R')Si(CₙH₂ₙ₋₁)
   - X =: Cl, Br
   - R' =: alkyl, such as, for example, methyl-, ethyl-, n-propyl-, i-propyl-, butyl-
   - R'=: cycloalkyl
   - n =: 1 - 20
e) Halogeno-organosilanes of the type X(R')₂Si(CₙH₂ₙ₊₁) and X(R')₂Si(CₙH₂ₙ₋₁)
   - X =: Cl, Br
   - R' =: alkyl, such as, for example, methyl-, ethyl-, n-propyl-, i-propyl-, butyl-
   - R'=: cycloalkyl
   - n =: 1 - 20
f) Organosilanes of the type (RO)₃Si(CH₂)ₘ-R'
   - R =: alkyl, such as methyl-, ethyl-, propyl-
   - m =: 0,1 - 20
   - R' =: methyl-, aryl (for example -C₆H₅, substituted phenyl radicals)
   -C₄F₉, OCF₂-CHF-CF₃, -C₆F₁₃, -O-CF₂-CHF₂
   -NH₂, -N₃, -SCN, -CH=CH₂, -NH-CH₂-CH₂-NH₂,
   -N-(CH₂-CH₂-NH₂)₂
   -OOC(CH₃)C=CH₂
   -OCH₂-CH(O)CH₂
   - NH-CO-N-CO-(CH₂)₅
   -NH-COO-CH₃, -NH-COO-CH₂-CH₃, -NH-(CH₂)₃Si(OR)₃
   -Sₓ-(CH₂)₃Si(OR)₃
   -SH
   -NR'R''R''' (R' = alkyl, aryl; R'' = H, alkyl, aryl; R''' = H, alkyl, aryl, benzyl, C₂H₄NR'''' R''''' where R'''' = H, alkyl and R''''' = H, alkyl)
g) Organosilanes of the type (R")ₓ(RO)_{y}Si(CH₂)ₘ-R'
   - R" =: alkyl x+y = 2
   - =: cycloalkyl x = 1,2
   y = 1,2
   m = 0,1 to 20
   - R' =: methyl-, aryl (for example -C₆H₅ , substituted phenyl radicals)
   -C₄F₉, -OCF₂-CHF-CF₃, -C₆F₁₃, -O-CF₂-CHF₂
   -NH₂, -N₃, -SCN, -CH=CH₂, -NH-CH₂-CH₂-NH₂,
   -N-(CH₂-CH₂-NH₂)₂
   -OOC(CH₃)C=CH₂
   -OCH₂-CH(O)CH₂
   -NH-CO-N-CO-(CH₂)₅
   -NH-COO-CH₃, -NH-COO-CH₂-CH₃, -NH-(CH₂)₃Si(OR)₃
   -Sₓ-(CH₂)₃Si(OR)₃
   -SH
   -NR'R''R''' (R' = alkyl, aryl; R'' = H, alkyl, aryl; R''' = H, alkyl, aryl, benzyl, C₂H₄NR'''' R''''' where R'''' = H, alkyl and
   - R''''' =: H, alkyl)
h) Halogeno-organosilanes of the type X₃Si(CH₂)ₘ-R'
   - X =: Cl, Br
   - m =: 0,1 - 20
   - R' =: methyl-, aryl (for example -C₆H₅, substituted phenyl radicals)
   -C₄F₉, -OCF₂-CHF-CF₃, -C₆F₁₃, -O-CF₂-CHF₂
   -NH₂, -N₃, -SCN, -CH=CH₂,
   -NH-CH₂-CH₂-NH₂
   -N-(CH₂-CH₂-NH₂)₂
   -OOC(CH₃)C=CH₂
   -OCH₂-CH(O)CH₂
   -NH-CO-N-CO-(CH₂)₅
   -NH-COO-CH₃, -NH-COO-CH₂-CH₃, -NH-(CH₂)₃Si(OR)₃
   -Sₓ-(CH₂)₃Si(OR)₃
   -SH
i) Halogeno-organosilanes of the type (R)X₂Si(CH₂)ₘ-R'
   - X =: Cl, Br
   - R =: alkyl, such as methyl,- ethyl-, propyl-
   - m =: 0,1 - 20
   - R' =: methyl-, aryl (e.g. -C₆H₅, substituted phenyl radicals)
   -C₄F₉, -OCF₂-CHF-CF₃, -C₆F₁₃, -O-CF₂-CHF₂
   -NH₂, -N₃, -SCN, -CH=CH₂, -NH-CH₂-CH₂-NH₂,
   -N-(CH₂-CH₂-NH₂)₂
   -OOC(CH₃)C=CH₂
   -OCH₂-CH(O)CH₂
   -NH-CO-N-CO-(CH₂)₅
   -NH-COO-CH₃, -NH-COO-CH₂-CH₃, -NH-(CH₂)₃Si(OR)₃,
   wherein R can be methyl-, ethyl-, propyl-, butyl-
   -Sₓ-(CH₂)₃Si(OR)₃, wherein R can be methyl-, ethyl-, propyl-, butyl-
   -SH
j) Halogeno-organosilanes of the type (R)₂XSi(CH₂)ₘ-R'
   - X =: Cl, Br
   - R =: alkyl
   - m =: 0,1 - 20
   - R' =: methyl-, aryl (e.g. -C₆H₅, substituted phenyl radicals)
   -C₄F₉, -OCF₂-CHF-CF₃, -C₆F₁₃, -O-CF₂-CHF₂
   -NH₂, -N₃, -SCN, -CH=CH₂, -NH-CH₂-CH₂-NH₂,
   -N-(CH₂-CH₂-NH₂)₂
   -OOC(CH₃)C=CH₂
   -OCH₂-CH(O)CH₂
   -NH-CO-N-CO-(CH₂)₅
   -NH-COO-CH₃, -NH-COO-CH₂-CH₃, -NH-(CH₂)₃Si(OR)₃
   -Sₓ-(CH₂)₃Si(OR)₃
   - SH
k) Silazanes of the type 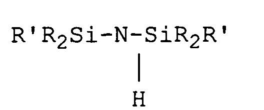
   - R =: alkyl, vinyl, aryl
   - R' =: alkyl, vinyl, aryl
l) Cyclic polysiloxanes of the type D 3, D 4, D 5, wherein D 3, D 4 and D 5 are understood as cyclic polysiloxanes with 3, 4 or 5 units of the type -O-Si(CH₃)₂-.E.g. octamethylcyclotetrasiloxane = D 4 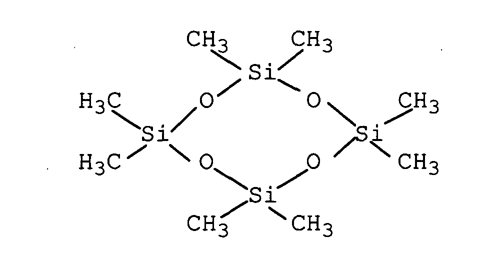
m) Polysiloxanes or silicone oils of the type 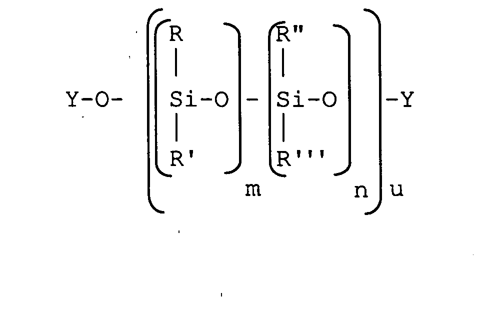
   - m =: 0,1,2,3, ... ∞
   - n =: 0,1,2,3, ... ∞
   - u =: 0,1,2,3, ... ∞
   Y=CH₃, H, CₙH₂ₙ₊₁ n=1-20
   Y=Si(CH₃)₃, Si(CH₃)₂H
   Si(CH₃)₂OH, Si(CH₃)₂(OCH₃),
   Si(CH₃)₂(CₙH₂ₙ₊₁) n=1-20
   - R =: alkyl, such as CₙH₂ₙ₊₁, wherein n = 1 to 20, aryl, such as phenyl und substituted phenyl radicals, (CH₂)ₙ-NH₂, H
   - R' =: alkyl, such as CₙH₂ₙ₊₁, wherein n = 1 to 20, aryl, such as phenyl- and substituted phenyl radicals, (CH₂)ₙ-NH₂, H
   - R'' =: alkyl, such as CₙH₂ₙ₊₁, wherein n = 1 to 20, aryl, such as phenyl- and substituted phenyl radicals, (CH₂)ₙ-NH₂, H
   - R''' =: alkyl, such as CₙH₂ₙ₊₁, wherein n = 1 to 20, aryl, such as phenyl und substituted phenyl radicals, (CH₂)ₙ-NH₂, H

The surface-modified zinc oxides according to the invention can be used for the preparation of cosmetics, in particular for the preparation of suncreen compositions.

The surface-modified zinc oxides according to the invention have the following advantages:

### Examples

### Analytical methods

The BET surface area is determined according to DIN 66131.

The transmission electron micrographs were obtained with a Hitachi transmission electron microscope, model H-75000-2. Approximately 500 to 600 aggregates were analysed by means of the CCD camera in the transmission electron microscope.

The variable F(shape) equals the quotient of the minimum to the maximum aggregate diameter. The variable F(circle) is calculated as F(circle) = 4π x average surface area)/2 (P), where P = circumference of the aggregates.

The variables F(shape) and F(circle) describe the deviation of a particle from a perfect circular shape. F(shape) and F(circle) are 1 for a perfect circular object. The lower the value, the further removed the object structure from the perfect circular shape.

The parameters are defined according to ASTM3849-89.

The surface properties are determined by large-area (1 cm²) XPS analysis (XPS = X-ray photoelectron spectroscopy), both in the original condition and after 30 minutes' surface erosion by ionic bombardment (5 keV argon ions). Fine structures of the oxygen signals are determined by Gaussian/Lorentzian curve analyses for oxygen.

One-percent aqueous solutions are used for the transmission measurements. Dispersion is performed by means of an ultrasonic instrument from Bandelin Elektronik. The sonication period is one minute. The measurements are taken using a Perkin Elmer Lambda 2 UV/Vis Spectrometer.

The bulk density was determined in accordance with DIN-ISO 787/XI.

### Examples

Figure 1 shows a flow diagram of the process according to the invention with the process stages and the incoming and outgoing mass flows.

There is: I = evaporation; II = nucleation; III = oxidation; IV = quenching; A = zinc oxide powder + inert gas; B = zinc vapour, water, (fuel gas); C = Zinc particles, water, (inert gas, fuel gas); D = zinc oxide particles, water, (inert gas); a = fuel gas, air/O₂ ; b = cooling (inert gas); c = air/O₂; d = cooling gas.

### Example 1:

Zinc powder (250 g/h, particle size =5 µm) is conveyed by means of a nitrogen stream (1.5 m³/h) into an evaporation zone, where a hydrogen/air flame (hydrogen: 4.25 m³/h, air: 8.40 m³/h, lambda = 0.82) is burning. The zinc powder is evaporated here. The reaction mixture consisting of zinc vapour, hydrogen, nitrogen and water is then cooled to a temperature of 850°C by the addition of 1 m³/h nitrogen. 5 m³/h oxidation air and 34 m³/h quench air are then added, whereby the reaction temperature falls to values below 400°C. The zinc oxide powder obtained is separated from the gas stream by filtration.

### Example 2

Same as Example 1, whereby the parameters are altered to the values shown in Table 1.

### Example 3 (comparative example)

Same as Example 1, except with an excess of air compared to oxygen in the evaporation zone. The parameters are altered to the values shown in Table 1.

### Example 4 (comparative example)

Same as Example 1, except with no nucleation zone, the temperature prior to oxidation does not fall below the boiling point of zinc. The parameters are altered to the values shown in Table 1.

The characterisation of the products obtained from these examples is shown in Table 2.

Evaluation of the image analysis reveals the clearest differences between the zinc oxide powders according to the invention and the prior art for the average surface area of the particles, the aggregate sizes and the shape factor F(circle).

XPS analyses were performed of the zinc oxide powders according to the invention from Examples 1 and 2. It was found that the moisture content as non-desorbable oxygen in the form of Zn-OH and Zn-OH₂ units is 55.5 % (Example 1) and 48.3 % (Example 2). The moisture is thus significantly higher for example in the Nanotek Zinc Oxide product from Nanophase Technologies.

Figure 2 shows a transmission electron micrograph of the powder according to the invention. Aggregates of nodular and acicular aggregates can clearly be seen.

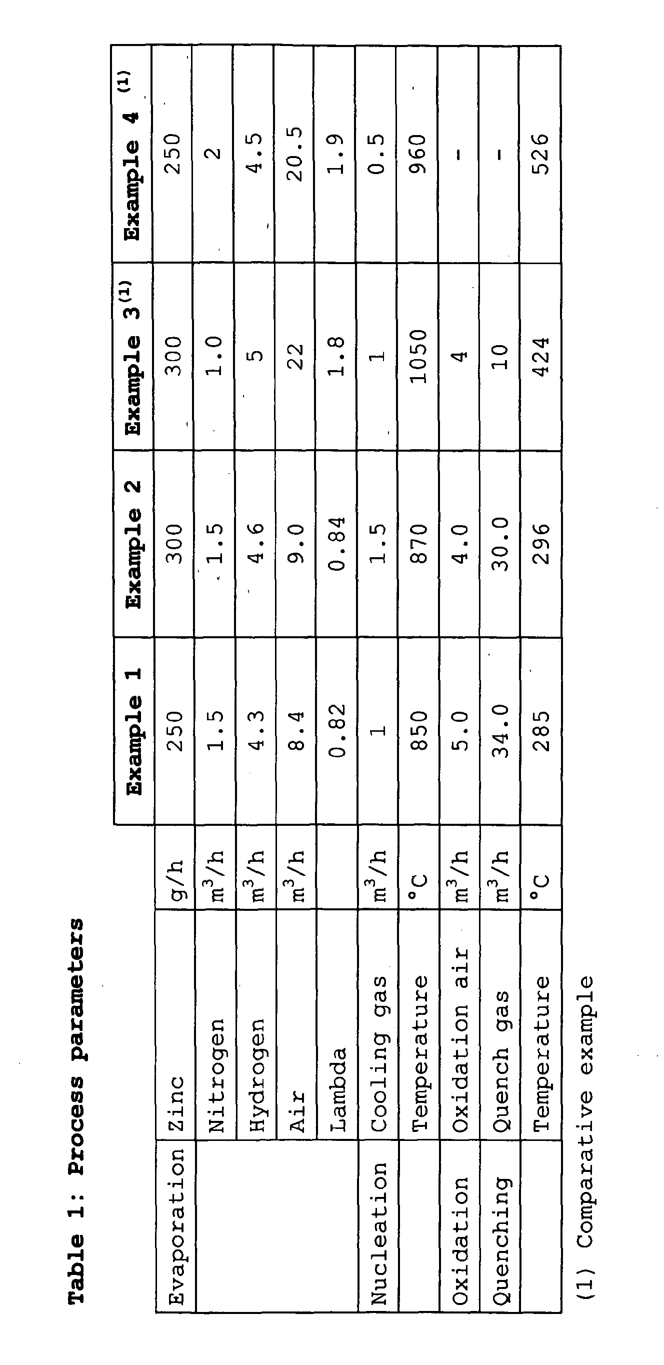

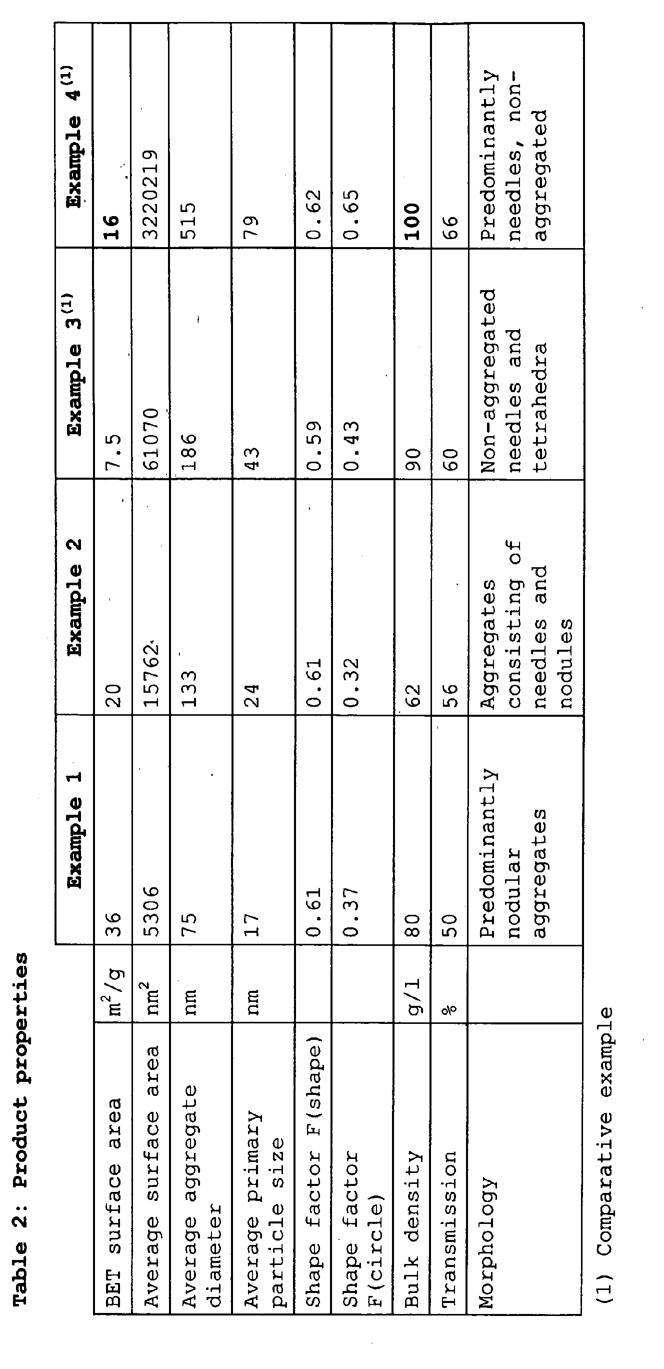

### Surface modification

For the surface modification, the zinc oxides are initially introduced into a mixer and, with intensive mixing, optionally first sprayed with water and then sprayed with the surface-modifying agent. When the spraying has ended, after-mixing can be carried out for a further 15 to 30 min, and then heat treatment for 1 to 4 h at 50 to 400°C.
The water employed can be acidified with an acid, for example hydrochloric acid, down to a pH of 7 to 1. The silanizing agent employed can be dissolved in a solvent, such as, for example, ethanol.

The hydrophilic zinc oxide employed is a zinc oxide which has a specific BET surface area of 21 m²/g and a pH of 6.9 according to (example .......)

**Table 1:**

| Surface modification of the zinc oxide preparation | | | |
|---|---|---|---|
| Example | 1 | 2 | 3 |
| Oxide | ZnO | ZnO | ZnO |
| Surface-modifying agent | octyltrimethoxysilane | octyltrimethoxysilane | polydimethylsiloxane |
| | | | |
| Parts of surface-modifying agent / 100 parts of oxide | 1.5 | 3 | 2 |
| | | | |
| Parts of H₂O / 100 parts of oxide | 0 | 0.2 | 0 |
| | | | |
| Temperature [°C] | 120 | 120 | 350 |
| | | | |
| Temperature time [h] | 2 | 2 | 2 |

**Table 2:**

| Physico-chemical data of the surface-modified products from table 1 | | | |
|---|---|---|---|
| Example | 1 | 2 | 3 |
| BET surface area [m²/g] | 18 | 18 | 17 |
| | | | |
| C content [%] | 0.6 | 0.9 | 0.6 |
| | | | |
| Loss on drying [%] | 0.1 | 0.1 | 0.2 |
| | | | |
| Loss on ignition [%] | 0.9 | 1.4 | 0.8 |
| | | | |
| pH | 6.5 | 6.8 | 7.3 |

**Use examples**

## Claims

1. Surface-modified zinc oxides, **characterized in that** they have the following physico-chemical characteristic data:
| | |
|---|---|
| BET surface areas | 18 ± 5 m²/g |
| C content | 0.5 to 1.0 wt.% |

2. Process for the preparation of the surface-modified zinc oxides according to claim 1, **characterized in that** the zinc oxides, optionally after spraying with water, are sprayed with the surface-modifying agent at room temperature and the mixture is then heat-treated at a temperature of 50 to 400°C over a period of 1 to 6 h.

3. Process for the preparation of the surface-modified zinc oxides according to claim 1, **characterized in that** the zinc oxides, optionally after spraying with water, are treated with the surface-modifying agent in vapour form and the mixture is then heat-treated at a temperature of 50 to 800°C over a period of 0.5 to 6 h.

4. Use of the surface-modified zinc oxides according to claim 1 for the preparation of cosmetics, in particular sunscreen compositions.
